# EUROPEAN PATENT SPECIFICATION

(11) **EP 3 512 593 B1**
(45) Date of publication and mention of the grant of the patent: **24.06.2020**
(21) Application number: 17851432.9
(22) Date of filing: 13.09.2017
(51) Int. Cl.: A61N 5/06, A61H 23/02, A61N 1/36

(54) **APPARATUS FOR INPHASE TREATMENT OF ATRAUMATIC MUSCULOSKELETAL PAIN**
VORRICHTUNG ZUR PHASENINTERNEN BEHANDLUNG VON ATRAUMATISCHEM MUSKEL-SKELETT-SCHMERZ
APPAREIL POUR LE TRAITEMENT EN PHASE D'UNE DOULEUR MUSCULO-SQUELETTIQUE ATRAUMATIQUE

(30) Priority: 15.09.2016 US 201662394907 P
(43) Date of publication of application: 24.07.2019
(73) Proprietor: Sense Technology Inc., Halifax, PA 17032 (US)
(72) Inventor: EVANS, Joseph M., Export, Pennsylvania 15632 (US)
(74) Representative: V.O.
(86) International application number: PCT/US2017/051302
(87) International publication number: WO 2018/052958

(56) References cited:
- KR-A- 20130 095 146
- US-A- 4 841 955
- US-A- 5 158 081
- US-A1- 2006 047 315
- US-A1- 2009 254 154
- US-A1- 2011 098 781
- US-A1- 2012 022 618
- US-A1- 2013 013 028
- US-A1- 2013 204 169
- US-A1- 2014 193 524
- US-A1- 2014 288 351
- US-A1- 2015 119 771
- US-A1- 2015 290 028
- US-A1- 2015 360 026

## Description

### BACKGROUND OF THE INVENTION

The incidence of low back and other pain continues to increase, with low back pain being the leading cause of disability in the world(Vassilaki & Hurwitz, 2014)(Werner & Cote, 2009 see Bibliography). In addition to the loss of quality of life for those who experience musculoskeletal pain the cost to both patients and society is significant and increasing:
- The annual cost of chronic pain in the United States, including healthcare expenses (direct medical costs), lost income, and lost productivity, is estimated to be $635 billion. This is significantly higher than the estimated annual costs in 2010, dollars of heart disease ($309 billion), cancer ($243 billion), and diabetes ($188 billion).
- Total estimated medical costs associated with back and neck pain, two of the commonest presentations of patients with chronic pain, increased by 65% between 1997 and 2005, to about $86 billion a year. Overall, pharmaceutical expenditures related to back and neck pain increased by 188% between 1997 and 2005, but costs associated with prescription narcotics rose by an astounding 423%.
- Nationally, the estimated annual direct medical cost of low back pain is $30 billion. In addition, the impact of back pain is $100-200 billion in decreased wages and lost productivity.
- Patients with chronic pain have more hospital admissions, longer hospital stays, and unnecessary trips to the emergency department.(Academy, 2011 see Bibliography)

Most back pain encountered by clinicians in their practice is referred to as nonspecific or as being of "unknown origin." Musculoskeletal pain resulting from trauma such as whiplash, repetitive strain injury and heavy lifting is relatively easy to understand; however, the cause and, therefore, best treatment for acute or chronic nonspecific musculoskeletal complaints is elusive.

The response to this problem by the allopathic profession has been to downplay the potential seriousness of the problem and to educate providers and patients.(Samanta, Kendall, & Samanta, 2003 see Bibliogrpahy) Other healing arts (referred to hereafter as "manual therapies") in the United States and elsewhere have developed terminology for the description of and procedures for treatment that are unique to each profession. Osteopaths refer to the problem as an "osteopathic lesion," chiropractors as a "subluxation,' physical therapists as "joint instability," acupuncturists as "trigger points," and massage therapists as "adhesions." Each of these professions has also developed what they consider to be unique approaches to therapy for these conditions;" manipulation," "adjustment," "muscle strengthening," "needling and acupressure," and "deep massage."

All techniques of manual therapy claim to reduce pain and even to provide a reversal of the underlying condition causing pain. However, despite numerous clinical trials comparing different approaches, there is no single methodology or technique that has achieved recognition as a primary or "first among equals" treatment. In fact there exist and are practiced hundreds of "named techniques" in the manual professions that have thousands of advocates who claim that their approach is superior to all others. This state of practice is testimonial to the fact that there is no universally recognized "best" treatment approach because there is no fundamental understanding of the cause of low back and other musculoskeletal pain.

All manual therapies have at least two features in common. The first is that individuals seeking treatment have defined themselves as patients. They have accepted that an authority in the treatment of their complaint can be of help. When they are examined by that authority, they are further reassured that they can be helped. This initial and vitally important step recruits the CNS (central nervous system) to assist in closing the gate that modulates the perception of pain, as predicted by the Gate Theory of Pain(Melzack & Wall, 1965 see Bibliography).

The second feature shared by all manual therapies is the application of proprioceptive afferent input to the CNS. The acupuncture needle, osteopathic manipulation, chiropractic adjustment, physical therapy mobilization and therapists massage all provide proprioceptive stimulation by mechanical pressure, impulse, impact, stretching and active and passive movement. These proprioceptive inputs presumably elicit responses from the Golgi tendon complex, muscle spindles and mechanoreceptors in joints, muscles, ligaments and fascia which participate in the restoration of proprioception to the CNS. This increase in proprioceptive input biases the summation of afferent inputs in the substantia gelatinosa toward domination by large fiber inputs, relieving the symptom of pain.

While all approaches to musculoskeletal therapy including simple encouragement provided to the patient by the allopathic profession are associated with improvement in the patients' perception of pain, the lack of a fundamental understanding of the cause of common low back and neck pain prevents the development of methods of treatment that are truly effective in reducing or eliminating the cause of pain. This is true whether the therapy consists of the adjustment, mobilization, massage, TENS, micro-current, heat, "laser" therapy and, perhaps the classic case, trigger point therapy where initially an injection of anesthetic was used then needling only and finally pressure alone.

No prior art for the treatment of the **underlying cause** of non-specific musculoskeletal pain exists because the underlying cause of such pain is unknown.

### PRIOR ART

The use of mineral supplements in the promotion of general health has a long history. Examples of the supply of minerals as ionic solutions are to be found in the form of trace minerals (http://www.traceminerais.com/products/liquid-tablet-minerals/concentrace-ionic-minerals) and (http://www.ancient-minerals.com). Searching for "mineral supplements" on the Amazon website returns 542 instances of trace mineral products. However, none of these products are formulated for the specific purpose of supporting the calcium pump in the function of muscle relaxation. Whereas the common formulation is either specific minerals like magnesium or a combination of minerals obtained from naturally occurring salt deposits, the current invention is designed to replicate the composition of electrolytes found in the tissues of the body with the intent of preferentially replacing any deficiency that may be found in the body.

The current invention teaches the topical application of a solution of electrolytes for the purpose of supporting the normal function of the calcium pump in its function of enabling muscle relaxation.

US Patent 8883830 teaches the use of topical formulations and methods of treating a migraines and/or cluster headaches, muscle sprains, muscle spasms, spasticity, tension headaches, tension related migraines and related conditions associated with muscle tension and pain with a therapeutically effective amount of an ergot alkaloid, skeletal muscle relaxant, serotonin agonist, combinations thereof, pharmaceutically acceptable salt thereof, prodrugs thereof or derivative thereof.

There is no teaching of the support of the Calcium pump nor of the use of a topical application of electrolytes for the purpose of enabling muscle relaxation. Although muscle tension and spasticity are mentioned as being associated with the conditions treated, the explanation of the underlying cause of muscle tension is cited as:
"Muscle spasm may occur as a result of direct soft tissue trauma with spasm of injured muscles. It may also arise as a consequence of spinal nerve root irritation from musculo-skeletal injury. Para spinal muscles are primarily affected in this situation, so called "cervical and lumbar sprains." Muscle spasm can manifest as a sudden involuntary contraction of one or more muscle groups and is usually an acute condition associated with muscle strain (partial tear of a muscle) or sprain (partial or complete rupture of a ligament). Spasticity is a state of increased muscular tone with exaggeration of the tendon reflexes from an upper motor neuron (brain or spinal cord) injury in which spinal inhibitory processes are suppressed or lost. The result is chronic, severe spasm of the muscles of the extremities hindering function and causing pain. Spasticity is often associated with illnesses such as multiple sclerosis, stroke and spinal cord injury. Tension headaches and tension-related migraines are a result of over activity of muscles of the scalp, forehead and neck."

This explanation relies on the elevation of nervous activity to initiate and maintain muscle spasm rather than the opposing view that the failure of the calcium pump is the true cause of the muscles inability to relax.

This patent may be most valuable as a representation of the thinking and theoretical basis for the current state of the art for the topical treatment of muscle spasm and associated pain. The stark differences between this approach and the invention illustrate the general lack of knowledge of the calcium pump and the role played by the calcium pump in the essential function of muscle relaxation.

Another embodiment of the current invention is the systemic application of a solution of electrolytes with the purpose of providing a systemic or generalized support of the calcium pump. Much of the existing art is focused on the ingestion of solutions of electrolytes for different purposes.

US Pat 4,042,684 teaches the replacement of sugar and electrolytes lost through vigorous exercise, there is no mention of enhanced muscle relaxation or elimination of pain. US Pat 4,322,407, teaches the replacement of electrolytes through use of an electrolytic drink for the purpose of replacing electrolytes and water. This patent provides a useful reference for comparing the specific formulas as it teaches that certain compositions are not readily absorbed by the body. The invention does not teach the use of electrolytes in supporting the calcium pump, ensuring that the environment necessary for the calcium pump to operate is maintained or supporting the ability of muscles to relax.

US Pat 4,839,347 teaches the treatment of dehydration in swine. No mention of muscle function etc.

US Pat 4,981,687 teaches the use of a composition of fluids comprised of water, sugar, electrolytes, and "non-toxic" substances (glycerol, pyruvate and caffeine) that are absorbed through the gastrointestinal tract to prevent loss of blood volume (during exercise) and serve as a source of energy. While containing electrolytes (metallic ions such as Ca, Mg etc.) the patent does not teach any mechanism for their use nor indicate that their ingestion may support muscle relaxation or muscle function in general.

The primary purpose of this patent appears to be as an enhancement to the previously patented solution known as Gatorade.

US Pat 5776952 teaches the application of a local anesthetic using a "carrier system" which overlies the local anesthetic in the area of pain.

The current invention teaches the use of adjusting instruments in conjunction with the topical application of a solution of electrolytes to enhance the effect of manual treatment of musculoskeletal complaints. Adjusting instruments have a history of art in themselves. US Pat 4,841,955, teaches a method and apparatus for the application of a reproducible impulse load to the body of a patient. This teaching is realized through the impulse trigger mechanism which requires the operator to apply a reproducible and known preload between the instrument and the patient which establishes a known initial force between the patient and the instrument prior to the application of the energy of the adjusting impulse. In contrast to other methods of adjusting patients, the energy of the impulse is precisely controlled and therefore reproducible,

US Patent 6702836 teaches the use of a manual settable component to select the frequency of the adjustment. It is not clear from the patent how this is accomplished or what the desired frequencies may be?

US Pat 7144417 teaches the use of a power supply regulating mechanism that purports to use AC current and voltage to provide a reproducible impulse to the body of the patient and couples this with a preload indicator and control means to provide indication of proper preload and impulse level.

US Pat 8641648 teaches the addition of a sensing element to the teaching of the prior US Pat 7144417.

None of the prior art of adjusting instruments teaches the application of manual therapy with adjusting instruments in support of the calcium pump or in conjunction with the topical application of a solution of electrolytes.

US Patent/ 5640978 teaches the treatment of pain due to periosteal-osseous injuries by irradiating the periosteal-osseous injury sites with low power laser energy to open lymphatic channels and remove the inflammatory substances so that the muscle spasm and withdrawal are alleviated and the referred pain is reduced. There is no mention of the calcium pump or the generation of ATP (Adenosine triphosphate) which is necessary for the operation of the calcium pump to enable relaxation of the muscle.

US Pat 8136531 teaches the use of multiple specific optical frequencies to treat musculoskeletal pain. While the generation of ATP as a result of laser irradiation is mentioned, there is no mention of the calcium pump or of muscle relaxation.

US Pat 8996131teaches the use of an implanted device for the purpose of exciting specific nerves through light stimulation and heat. There is no mention of the calcium pump or ATP.
"Chronic or recurrent pain affects 20-25% of the U.S. population, and it leads to approximately $100 billion in health care costs each year. The lost productivity due to pain is estimated at approximately $50 billion per year in the U.S. (low back pain is alone responsible for about a third of this figure). Traditional pain treatments include drugs (e.g., opioids (the world-wide market size for opioids (e.g., morphine and hydrocodone) is approximately $36 billion), anti-convulsants, anti-depressants, epidurais/anesthetics), surgery (e.g., disk surgery, nerve cutting), cognitive/behavioral (e.g., biophsychosocial approach, relaxation/biofeedback, placebo), and physical therapy. Other non-traditional approaches to pain management include acupuncture, ultrasound, and LLLT (low-level light therapy). The rule of thumb for leading pain researchers is that almost every major pain treatment creates about a 50% reduction in pain for 30-40% of patients (there is generally no good way to identify who will respond to a given treatment). Many drugs, particularly opioids, carry significant side effects and can become addictive. Depending on the study, 10-49% of back surgery patients are worse after the surgery ("failed back surgery syndrome").

US Pat 9061135 teaches a refinement of US Pat 8996131.

None of the prior art cited here teaches the use of laser or low level light stimulation to support the function of the calcium pump or to generate ATP in conjunction with mechanical stimulation to provide energy to support the function of the calcium pump.

### BRIEF DESCRIPTION OF THE DRAWINGS

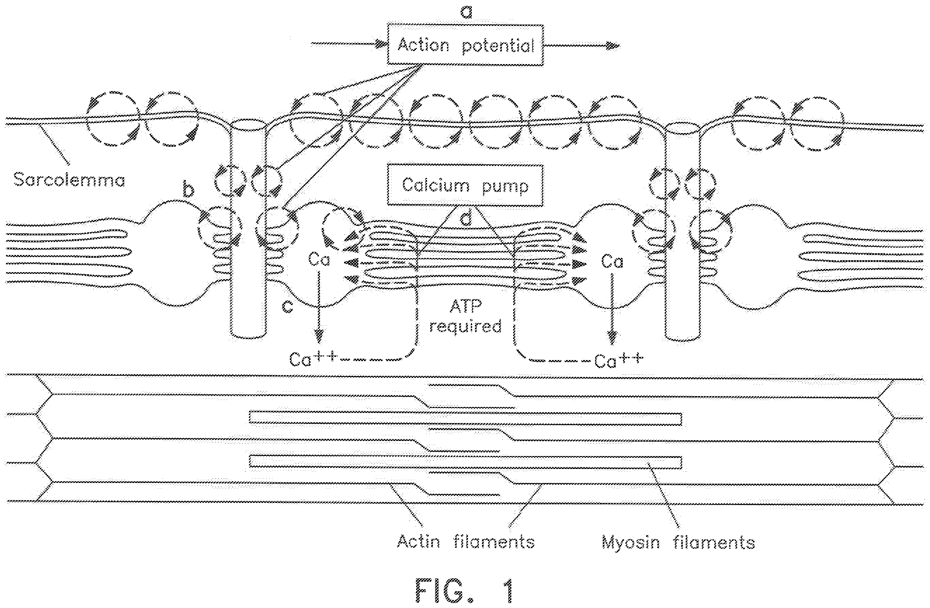

Figure 1. Stages of Normal Muscle Function: Fig. 1a Muscle Fiber Depolarization Initiated by Motor Nerve; Fig. 1b. Muscle Fiber Depolarization Opens Calcium Channels in Sarcoplasmic Reticulum; Fig 1c. Calcium Ions Stored in Sarcoplasmic Reticulum Infuse Muscle Fiber Causing Muscle Contraction; Fig 1d. Calcium Pump Returns Calcium Ions to Sarcoplasmic Reticulum Allowing Muscle to Relax (Adapted from "Textbook of Medical Physiology" Guyton and Hall Edition 12, see Bibliography)
Figure 2. Normal Cycle of Muscle Contraction and Relaxation Produces Lymphatic Flow as well as Proprioceptive Input to the Central Nervous System
Figure 3. Failure of calcium pump results in inability of muscle to relax resulting in compromise of lymphatic system and perception of pain.
Figure 4 consists of Figures 4A-4C, Figure 4A shows a perspective view of the Impulse head (1) without the laser attachment. Figure 4B shows a perspective view of the Laser attachment (2). Figure 4C shows a perspective view of the impulse head (1) with the Laser attachment (2) in place.
Figure 5 consists of Figures 5A and 5B. Figure 5A shows a cross section view of the impulse head with the Laser attachment in place. Figure 5B shows a cross section of the laser attachment.
Figure 6 consists of Figure 6A and 6B. Figure 6A shows the top side of the home use instrument which is designed to provide a comfortable and secure handhold for application of the instrument. Figure 6B shows the underside of the instrument which consists of a soft transparent material which allows the underside to apply motion therapy as well as light therapy to the patient in conjunction with or independently of the application of lotion therapy.
Figure 7 shows a cross-section of the home use instrument.

### DETAILED DESCRIPTION OF THE INVENTION

in the absence of a fundamental understanding of the true cause of common low back and neck pain (usually referred to as "pain of unknown origin) there can be no invention of improved therapy or methodology for the improved treatment of such pain. Therefore, in order to create a truly useful invention, one that would be of benefit to therapists by providing improved patient results through the practice of the invention and benefit the patient through faster reduction in pain as well as reduction in relapses and improved general health, a new approach to the understanding of common musculoskeletal pain is required that identifies the true cause of such pain. The present invention is dependent on this approach and the invention cannot exist without this understanding.

In order to develop an understanding of muscle dysfunction that results in pain, it is necessary to first understand normal pain-free muscle function. Fortunately, the fundamental physiology of normal muscle function is described in current physiology texts(Hall & Guyton, 2011 see Bibliography) and is generally recognized as an accurate portrayal of the contraction and, most importantly, the relaxation of muscle fibers. According to these texts, the cause of muscle fiber contraction is the release of calcium ions from the sarcoplasmic reticulum (a complex sac that interpenetrates the muscle) causes contraction of the myofibrils which results in contraction of the muscle fiber. Subsequent relaxation of the muscle fiber is enabled by the ATP energized calcium pump which returns calcium ions to the sarcoplasmic reticulum where they are available for the initiation of the next muscle contraction.

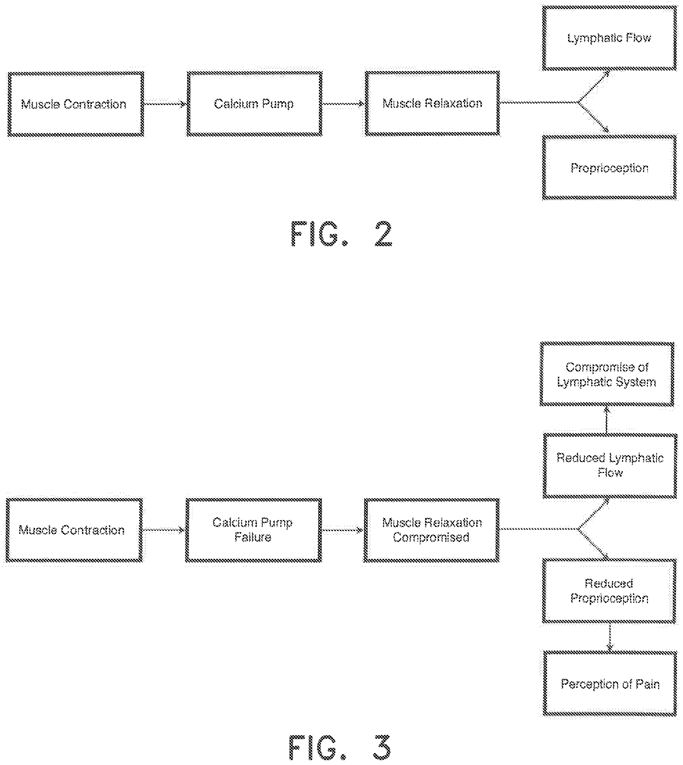

As a result of and coincident with normal muscle contraction and relaxation, sensors within the muscle (Golgi tendon complex and muscle spindles) continuously send information to the central nervous system (CNS) regarding muscle length, speed of contraction and force. These signals are collectively referred to as proprioception. In addition, the contraction and relaxation of the muscles of the body play a significant part in the maintenance of lymphatic drainage of the waste products of cellular metabolism from the body. See Figure 2.

If the calcium pump malfunctions, the muscle cannot relax. Interruption of the normal contraction and relaxation cycle of the muscle in turn interrupts the normal flow of proprioceptive input to the CNS as well as the normal flow of lymphatic fluid. Disruption of proprioceptive input to the CNS is perceived as pain and disruption of the lymphatic flow can result in metabolic failure at the cellular level. See Figure 3.

Therefore the cause of atraumatic muscle pain is:
- An acute stage consisting of an initiating event wherein a portion of muscle mass in a normal contractile state is prevented from extending or relaxing due to a local failure of the calcium pump which results in the inability of the muscle to relax. The inability of the muscle to relax results in the perception of pain.
- If the muscle dysfunction is not immediately corrected, the initial muscle dysfunction will result in chronic compromise of the lymphatic system resulting in potentially serious and poorly understood consequences for the health of the individual. Such consequences may include but are not limited to: localized edema, decreased blood flow, production of abnormal products of cellular metabolism and other potentially serious health effects.

Calcium ions are the primary mediator of contraction itself and therefore must be available in sufficient quantity for normal muscle contraction and relaxation. The release of calcium into the muscle fiber causes contraction of the myofibrils which results in contraction of the muscle fiber. Subsequent relaxation of the muscle fiber is enabled by the calcium pump which returns calcium ions to the sarcoplasmic reticulum where they are available for the initiation of the next muscle contraction. The function of the calcium pump is dependent upon the availability of adenosine triphosphate (ATP) which supplies the pumping energy. A failure of the calcium pump would account for the inability of a muscle fiber to relax. Such a failure could be due to a reduction in the synthesis of ATP for continued cycling of the ATP powered calcium pump.

ATP synthesis can be restricted by a pH that is either too acidic or basic. Other mechanisms may also account for reduced levels of ATP that would compromise the calcium pump. It is well known that minimum levels of potassium ion concentration are necessary to allow contracted muscle to relax(Hall & Guyton, 2011 see Bibliography). Other metallic ions such as sodium, potassium, magnesium, calcium and phosphorous play important roles not only as mediators of membrane depolarization and myofibril contraction, but as necessary modulators of the of the creation and utilization of ATP. Metallic ion concentrations may exhibit abnormal local concentrations which reduce the availability of ATP leading to the inability of small localized muscle volumes or entire muscles to relax.

Therefore, in order to prevent the failure of the calcium pump, an adequate supply of Ca²⁺ ions as well as ATP must be maintained in the cellular interstitial space as well as the sarcoplasmic reticulum.

All techniques of manual therapy claim to successfully reduce pain and even to provide a reversal of the underlying condition causing pain. This is true for the adjustment, mobilization, massage, TENS, micro-current, heat, "laser" therapy and, perhaps the classic case, trigger point therapy where initially an injection of anesthetic was used then needling only and finally pressure alone. One must speculate that each and every one of these approaches activates the same or a similar underlying process. Finding that the same neurochemical process is associated with each of these techniques would suggest that each individual technique may produce similar therapeutic effects through the same mechanism. Indeed there appears to be such a process. It has been known for at least thirty years that electrical current stimulates ATP(Cheng et al., 1982 see Bibliography). More recently, the existence of a reversible chemomechanical coupling mechanism associated with ATP synthesis that generates rotary motion at the molecular level has been demonstrated.(Montemagno & Bachand, 1999; Noji & Yoshida, 2001; Watanabe & Noji, 2013 see Bibliography) Direct evidence for the chemical synthesis of ATP as a result of mechanical action is presented by Itoh et.al.(Itoh et al., 2004 see Bibliography) ATP release from mast cells during needling, application of heat and exposure to light stimulation has been demonstrated by Wang et.al.(Wang et al., 2013 see Bibliography) If therapeutic stimulation of all kinds affects a release of ATP and ATP is necessary for muscle relaxation then the correlation between stimulation of cellular ATP by these various methods and pain relief may be causal in nature. That is, the production of ATP by these methods of therapy may be a primary route leading to pain reduction through the restoration or enhancement of the calcium pump.

Assuming that the stimulation of ATP by all types of manual therapy is a primary mechanism for the restoration of normal neuromuscular function at the cellular level leading to reduction of pain, it would be apparent to one skilled in the art of pain treatment that the simple combination of two or more therapies might be beneficial. That is, rather than applying only one manual therapy, the application of two different types of manual therapy would be expected to provide additional benefits because the recruitment of additional ATP through separate channels would lead to more efficient generation of ATP. Rather than simply combine two therapies additively with the expectation that the combination will result in a more effective pain reduction therapy, the invention utilizes a novel and unique method of combining two or more therapies so that the therapies may be applied simultaneously and, more importantly, in phase.

A mechanical impulse device such as the multiple impulse adjusting instrument described in US Pat 4,841,955 and 5,662,122 can serve as the foundation for the creation of a new instrument that combines photonic (light) and optionally electrical (tens or micro-current) stimulation with proprioceptive (mechanical impulse) stimulation in a novel way to obtain more effective pain reduction more quickly than the application of these same methods as separate treatment modalities. Simultaneous application of any two or even all of these methods may be achieved by the use of a separate effector head which is attached to and energized by the attachment mechanism itself. Not only can the application of two or more modalities be achieved simultaneously but their application can be synchronized to be "in phase" with one another. In phase means that the initiation, rate of application and peak amplitude of each separate treatment modality may be controlled to coincide to achieve maximum effect at the same time in the treatment cycle.

In order to create an instrument that is capable of combining the three modes of therapy and control the phase of each, we start with a modification of the holder of the interchangeable patient contact prongs of the multiple impulse adjusting instrument described in US Pat 5,662,122. adding a positive Voltage and ground connection to this holder to provide power to the patient probe used for the simultaneous application of additional therapeutic light and optionally electrical modalities. A suitably sized and configured mechanism for the connection of the supplied Voltage to the patient probe (such as an audio plug DigiKey part no SC1326-ND) will provide a suitable electrical connection between the impulse adjusting instrument and the new patient contact probe. The electrical power provided to the patient contact probe will provide the energy needed for the application of the light and optionally electrical therapy.

The timing of the application of the light therapy and optionally electrical therapy is controlled to be in phase with the application of proprioceptive therapy provided by the multiple impulse adjusting head by means of a control mechanism consisting of a means of sensing the resistance between the patient contact probe and the patient (such as a force sensor) or the movement of the patient contact probe (such as an accelerometer). The additional therapy of light and optionally electrical stimulation may be added to the application of proprioceptive therapy at the precise time in the impulsive cycle that will provide the most effective effect of the combined therapies.

While the above procedure will result in pain relief due to muscle relaxation independent of other factors, the preferred embodiment of the invention is to apply the combination of proprioceptive, light and electrical therapies in combination with a solution of metallic ions (see US Provisional Patent Application No. 62/204,622). The combination of therapies such as massage, manipulation, adjustment, ultrasound, transcutaneous electrical stimulation, light stimulation etc. are known to have the common result of stimulating the synthesis of ATP enhancing the efficiency and effectiveness of pain relief. Manual or methods of proprioceptive stimulation have the additional advantage of flushing of the lymphatic drainage of the involved musculature removing irritants generated by the effects of muscle dysfunction and normalization of metallic ion concentrations within the intracellular spaces of the muscular tissue.

The current invention teaches the maintenance and enhancement of the calcium pump through the phased application of multiple distinct therapeutic modalities such as the combination of proprioception, light and electrical stimulation, a concept that has not until now been associated with muscle pain, especially the inability of muscle to relax due to the failure of the calcium pump. The purpose of the current invention is to reduce or eliminate the pain associated with the failure of the calcium pump and the inability of the muscles to relax and to maintain and enhance general muscle function and metabolic health.

US provisional patent application 62/204,622 teaches the use of a lotion or salve containing electrolytes necessary for the maintenance of normal muscle function. While the present invention will efficiently reduce pain and inflammation due to failure of the calcium pump, optimum results will be obtained when the invention comprising proprioceptive stimulation and phased light and optionally electrical stimulation is applied in concert with said lotion. The additional effectiveness is obtained by means of the light stimulation interacting with the molecular structure of the elements of the lotion, thereby increasing the molecular energy of the lotion and enabling enhanced diffusion of the beneficial constituents of the lotion to the site of muscle impairment.

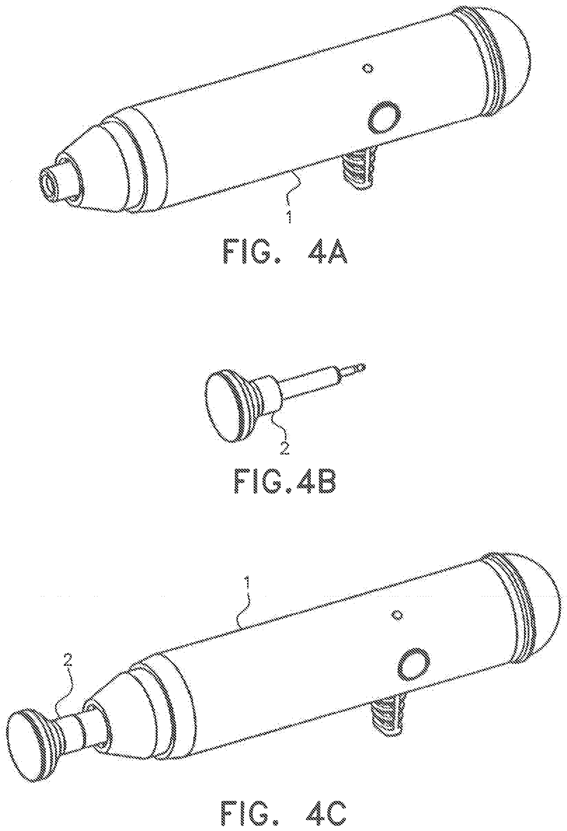

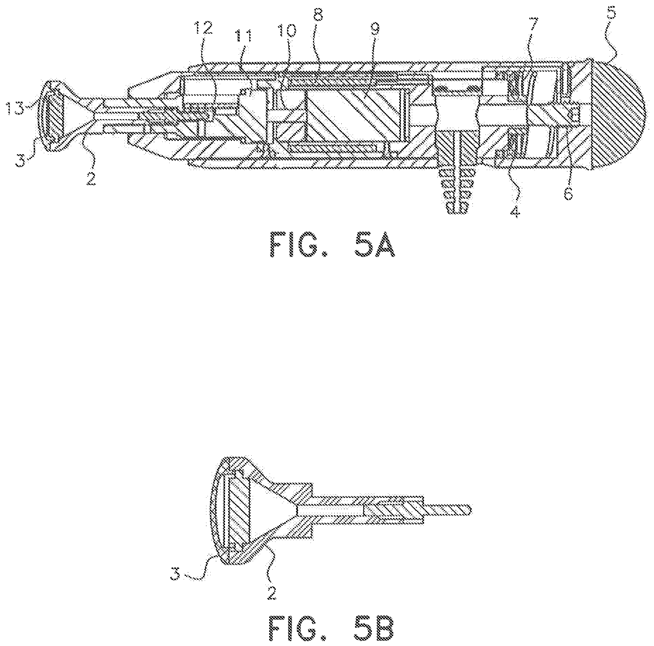

In a first preferred embodiment, the invention may be realized in the manner described above, comprising the combination of an instrument designed to provide multiple impulse proprioceptive stimulation (mechanical displacement of the involved muscle mass of varying force, depth and frequency obtained with mechanical patient contact probes, see US Pat. 4,841,955, Chiropractic Device and US Pat. 5,435,813) and a separate means for the addition and synchronism of light and/or electrical stimulation wherein the energy for the light or electrical stimulation is drawn from the instrument or from a battery attached to the light or electrical stimulation apparatus. This embodiment is shown in Figures 4 and 5A and B.

The clinician places the transparent face of the Laser attachment Fig. 5(3) at the desired point on the patient and compresses the preload spring Fig 5(4) by applying force to the end cushion Fig 5(5). This action forces the magnetic rod (6) into the preload sense coil Fig 5(7). The resulting change in inductance of the preload sense coil Fig 5(7) is sensed by the impulse head control circuitry (not shown) as a change in frequency of a "tank circuit" of which the preload sense coil Fig 5(7) is a part. When the compression of the preload sensor composed of the preload spring Fig 5(4), the magnetic rod Fig 5(6) and the preload sense coil Fig 5(7) reaches a predetermined force against the patient as indicated by a predetermined change in the inductance of the preload sense coil Fig 5(7), the impulse head solenoid coil Fig 5(8) is energized by the control circuitry (not shown). The impulse provided by the impulse head solenoid energy will therefore always begin when the preload force against the patient is the same providing a basis for a repeatable consistent therapeutic mechanical impulse to the patient. Once the impulse is initiated by the compression of the impulse against the patient, a predetermined, measured amount of energy is provided to the impulse head solenoid coil Fig 5(8) which determines the average force applied to the patient during the impulse. The lowest energy level results in an average force of approximately five pounds force and the highest force results in an average force of approximately thirty-five pounds force. The above describes the function of the impulse head of US Patent Number 4,841,955 and US Patent Number 5,662,122.

When the purely mechanical patient contact probes are replaced with the active light emitting patient contact Fig.5(2), a second therapeutic modality is introduced. When the impulse head solenoid coil Fig. 5(8) is energized, the energy of the solenoid results in the movement of the solenoid armature Fig. 5(9) toward the patient causing the drive rod Fig. 5(10) to move toward the patient as well. The drive rod Fig. 5(10) is in contact with the impulse head attachment holder Fig. 5(11) which also moves toward the patient causing the Laser attachment to transfer force to the patient while compressing the underlying tissue at the point of patient contact. A sensor means (accelerometer) on the Laser attachment circuit board Fig. 5(12)) or force sensor (not shown) is used to measure the motion of the Laser attachment Fig. 5(2) that results from the movement. A light emitting source (laser diode or LED) mounted on the Laser attachment circuit board Fig. 5(13) is energized when the desired point in the movement toward the patient (which is analogous to the amount of compression of the tissue underneath the Laser attachment Fig. 5(2) is reached. Thus the area underneath the Laser attachment Fig. 5(2) may be flooded with photo-stimulus at any point in the mechanical impulse provided to the patient in order to obtain the most therapeutic effect.

In addition to timing the application of the laser therapy for maximum effect, the Laser attachment circuit board Fig. 5 (13) may contain a multitude of light sources that vary in frequency from far infra-red to white light. In addition, the use of separate Laser attachments Fig. 5(2) with different light source frequencies will result in the ability of the clinician to tailor the light frequency to obtain maximum clinical effect.

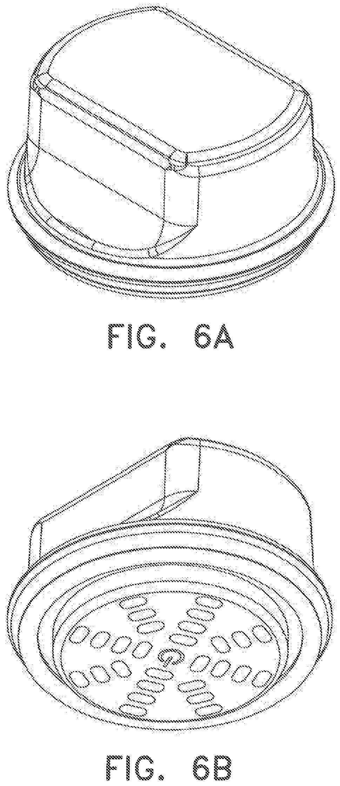

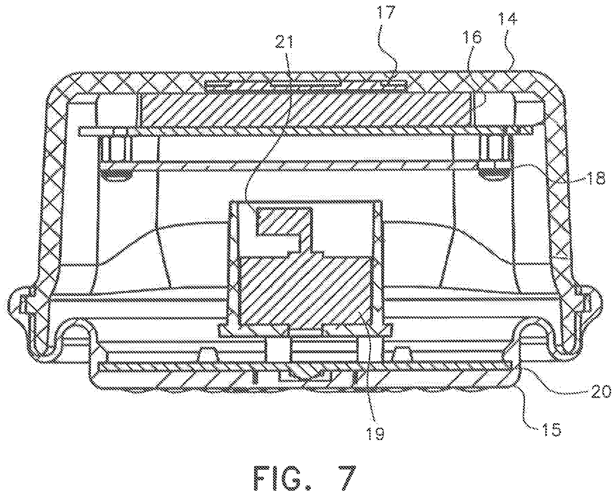

In a second preferred embodiment, the invention may be realized by the combination of elements as shown in Figures 6A, 6B and 7 where a proprioceptive stimulus is provided by a motor with an eccentric weight fixed to the motor shaft as a home use instrument.

The home use instrument housing Fig. 7(14) provides a convenient way to hold the instrument and also encloses the electromechanical parts of the instrument. The translucent patient contact Fig. 7(15) is attached to the bottom of the housing Fig. 7(13) forming a flexible and watertight means of providing both mechanical and photonics stimulation to the patient. The battery Fig. 7(16) provides the energy for instrument operation is charged via the radio frequency coil Fig. 7(17). The upper circuit board Fig. 7(18) houses the battery charging circuit and the control means for programming the operation of the instrument including radio communication with an iPhone app, speed of the motor Fig. 7(19) and excitation of the light source or sources on the lower printed circuit board Fig. 7(20).

During operation of the home use instrument, the motor Fig. 7(19) turns a weight Fig. 7(20) mounted eccentrically on the motor shaft which causes the motor Fig. 7(19) and the lower circuit board Fig.7(20) to which it is attached, to oscillate transmitting mechanical force to the patient to provide proprioceptive stimulation to the central nervous system of the patient. Simultaneously, the light sources are selectively energized to provide photonic stimulation in phase with or in opposition to the mechanical stimulation provided through the patient contact Fig. 7(15).

## Claims

1. Apparatus for simultaneous in-phase neuromusculoskeletal treatment comprising:
an end effector, a housing, a source of electromagnetic radiation and a source of energy;
the end effector consisting of a patient contact element comprised of a material transparent to electromagnetic radiation with frequencies of interest for treatment of neuromusculoskeletal complaints, said contact element is mounted in the housing with the source of said electromagnetic radiation mounted behind the patient contact element and the source of energy for the operation of the source of electromagnetic radiation, said housing having an end opposite the patient contact element configured to provide both mechanical and electrical coupling to a single or multiple impulse therapy instrument designed to provide mechanical impulses to the patient for the relief of neuromuscular complaints such as musculoskeletal pain due to joint subluxation, restricted joint mobility, myofacial spasm and ligamentous strain,
a controller to synchronize the radiation therapy with the mechanical impulse comprising:
a microprocessor; a switch to establish the initiation of the mechanical impulse coupled with the microprocessor to initiate and control the length of single or multiple radiant pulses at any point within the mechanical impulse or a sensor such as an accelerometer or force sensor which monitors the mechanical impulse and is coupled to the microprocessor which is programmed to control the initiation and length of single or multiple radiant pulses at any point within the mechanical impulse.

2. Apparatus as claimed in claim 1, wherein the frequencies of interest for treatment of neuromusculoskeletal complaints are in the range of ultraviolet to far infrared.

3. Apparatus as claimed in claim 1, wherein the source of radiation consists of a single frequency.

4. Apparatus as claimed in claim 1, wherein the source of radiation consists of a plurality of frequencies.

5. Apparatus as claimed in claim 1, wherein the end effector is comprised of a plurality of patient contact elements.

6. Apparatus as claimed in claim 3, wherein the controller allows for independent control of a plurality of patient contact elements.

7. Apparatus as claimed in claim 1, wherein the end effector is selected from:
a Laser Adaptor,
a Laser,
a Cold Laser, or
an LED.

## Patentansprüche

1. Vorrichtung zur gleichzeitigen phasengleichen Behandlung eines Neuromuskuloskeletts, umfassend:
einen Endeffektor, ein Gehäuse, eine Quelle elektromagnetischer Strahlung und eine Energiequelle; wobei
der Endeffektor aus einem Patientenkontaktelement besteht, das aus einem für elektromagnetische Strahlung zur Behandlung von neuromuskuloskelettalen Beschwerden relevanten Frequenzen durchlässigem Material besteht, wobei das Kontaktelement im Gehäuse montiert ist, wobei die Quelle elektromagnetischer Strahlung hinter dem Patientenkontaktelement und der Energiequelle für den Betrieb der Quelle elektromagnetischer Strahlung montiert ist, wobei das Gehäuse ein dem Patientenkontaktelement gegenüberliegendes Ende aufweist, das dazu ausgelegt ist, sowohl eine mechanische als auch eine elektrische Kopplung mit einem Einzel- oder Mehrfachimpulstherapieinstrument bereitzustellen, das dazu ausgelegt ist, dem Patienten mechanische Impulse zur Linderung von neuromuskulären Beschwerden wie muskuloskelettalen Schmerzen aufgrund von Subluxation der Gelenke, eingeschränkter Beweglichkeit der Gelenke, myofazialem Krampf und Bandbelastung bereitzustellen;
eine Steuerung zum Synchronisieren der Strahlentherapie mit dem mechanischen Impuls, umfassend:
einen Mikroprozessor;
einen Schalter zum Herstellen der Einleitung des mechanischen Impulses, verbunden mit dem Mikroprozessor zum Einleiten und Steuern der Länge einzelner oder mehrerer Strahlungsimpulse mit einem beliebigen Punkt innerhalb des mechanischen Impulses oder einem Sensor wie einem Beschleunigungsmesser oder Kraftsensor, der den mechanischen Impuls überwacht und mit dem Mikroprozessor gekoppelt ist, der so programmiert ist, dass er die Einleitung und Länge einzelner oder mehrerer Strahlungsimpulse an jedem Punkt innerhalb des mechanischen Impulses steuert.

2. Vorrichtung nach Anspruch 1, wobei die interessierenden Frequenzen zur Behandlung von neuromuskuloskelettalen Beschwerden im Bereich von ultraviolettem bis fernem Infrarot liegen.

3. Vorrichtung nach Anspruch 1, wobei die Strahlungsquelle aus einer einzelnen Frequenz besteht.

4. Vorrichtung nach Anspruch 1, wobei die Strahlungsquelle aus mehreren Frequenzen besteht.

5. Vorrichtung nach Anspruch 1, wobei der Endeffektor aus mehreren Patientenkontaktelementen besteht.

6. Vorrichtung nach Anspruch 3, wobei die Steuerung eine unabhängige Steuerung einer Vielzahl von Patientenkontaktelementen ermöglicht.

7. Vorrichtung nach Anspruch 1, wobei der Endeffektor ausgewählt ist aus:
einem Laseradapter,
einem Laser,
einem kalten Laser oder
einer LED.

## Revendications

1. Appareil de traitement neuromusculosquelettique en phase simultané comprenant :
un organe terminal effecteur, un boîtier, une source de rayonnement électromagnétique et une source d'énergie ;
l'organe terminal effecteur consistant en un élément de contact avec le patient constitué d'un matériau transparent au rayonnement électromagnétique à des fréquences d'intérêt pour le traitement de troubles neuromusculosquelettiques, ledit élément de contact étant monté dans le boîtier avec la source dudit rayonnement électromagnétique montée derrière l'élément de contact avec le patient et la source d'énergie pour le fonctionnement de la source de rayonnement électromagnétique, ledit boîtier ayant une extrémité opposée à l'élément de contact avec le patient configurée pour assurer un couplage aussi bien mécanique qu'électrique à un instrument de thérapie par impulsions uniques ou multiples conçu pour appliquer des impulsions mécaniques au patient pour le soulagement de troubles neuromusculaires tels qu'une douleur musculosquelettique due à une subluxation articulaire, une mobilité articulaire restreinte, un spasme myofascial et une entorse ligamentaire,
un dispositif de commande pour synchroniser la radiothérapie avec l'impulsion mécanique comprenant :
un microprocesseur ;
un interrupteur pour établir le déclenchement de l'impulsion mécanique couplé au microprocesseur afin de déclencher et de commander la durée d'impulsions de rayonnement uniques ou multiples à tout instant au cours de l'impulsion mécanique ou
un capteur tel qu'un accéléromètre ou un capteur de force qui surveille l'impulsion mécanique et est couplé au microprocesseur qui est programmé pour commander le déclenchement et la durée d'impulsions de rayonnement uniques ou multiples à tout instant au cours de l'impulsion mécanique.

2. Appareil selon la revendication 1, dans lequel les fréquences d'intérêt pour le traitement de troubles neuromusculosquelettiques sont dans la gamme de l'ultraviolet à l'infrarouge lointain.

3. Appareil selon la revendication 1, dans lequel la source de rayonnement est composée d'une seule fréquence.

4. Appareil selon la revendication 1, dans lequel la source de rayonnement est composée d'une pluralité de fréquences.

5. Appareil selon la revendication 1, dans lequel l'organe terminal effecteur est constitué d'une pluralité d'éléments de contact avec le patient.

6. Appareil selon la revendication 3, dans lequel le dispositif de commande permet une commande indépendante d'une pluralité d'éléments de contact avec le patient.

7. Appareil selon la revendication 1, dans lequel l'organe terminal effecteur est choisi parmi :
un adaptateur laser,
un laser,
un laser de faible puissance, ou
une DEL.
